# EUROPÄISCHE PATENTANMELDUNG

(11) **EP 4 613 361 A1**
(43) Veröffentlichungstag der Anmeldung: **10.09.2025**
(21) Anmeldenummer: 25160982.2
(22) Anmeldetag: 28.02.2025
(51) Int. Cl.: B01D 61/12, A61M 1/16, C02F 1/00, C02F 1/44

(54) **VERFAHREN ZUM BETREIBEN EINER UMKEHROSMOSEANLAGE**

(30) Priorität: 06.03.2024 DE 102024106402
(71) Anmelder: B. Braun Avitum AG, 34212 Melsungen (DE)
(72) Erfinder: LIEB, Nino, 34212 Melsungen (DE); ODERNHEIMER, Philipp, 34212 Melsungen (DE)
(74) Vertreter: Tergau & Walkenhorst Intellectual Property GmbH

(57) **Zusammenfassung**

Verfahren zum Betreiben einer Umkehrosmoseanlage (100), welche wenigstens eine Druckerhöhungspumpe (108), wenigstens ein Membranmodul (110) und eine Ringleitung (102) mit wenigstens einer Abnahmestelle (103) umfasst, mit den Schritten
a) Selektieren wenigstens eines anlagenspezifischen Parameters (D, Kp, Ti) der Umkehrosmoseanlage (100) und Definieren einer optimierten Systemreaktion der Umkehrosmoseanlage (100) in Abhängigkeit von dem wenigstens einen anlagenspezifischen Parameter (D, Kp, Ti) der Umkehrosmoseanlage;
b) iteratives Verändern des wenigstens einen anlagenspezifischen Parameters (D, Kp, Ti) der Umkehrosmoseanlage (100);
c) Messen der Systemreaktion der Umkehrosmoseanlage (100) auf den wenigstens einen veränderten anlagenspezifischen Parameter (D, Kp, Ti) der Umkehrosmoseanlage;
d) optionales Anpassen des Veränderns des wenigstens einen anlagenspezifischen Parameters (D, Kp, Ti) der Umkehrosmoseanlage (100), um eine Systemreaktion zu erreichen, welche näher an der optimierten Systemreaktion liegt;
e) bei Erreichen der optimierten Systemreaktion innerhalb eines vorgegebenen Schwellenbereiches, Festlegen wenigstens eines optimierten Betriebsparameters (D, Kp, Ti) und/oder Schwellenwertes (PS, J);
f) Betreiben der Umkehrosmoseanlage (100) mit diesem wenigstens einen optimierten Betriebsparameter und/oder Schwellenwert (PS, J).

## Beschreibung

Die Erfindung betrifft ein Verfahren zum Betreiben einer Umkehrosmoseanlage nach dem Oberbegriff von Anspruch 1.

Umkehrosmoseanlagen werden im medizinischen Bereich verwendet, um Wasser z.B. für Dialysegeräte herzustellen, welche dann für eine Dialysebehandlung durchführen. Während der Dialysebehandlung kommen Patienten mit einer großen Menge an Dialysierflüssigkeit in Kontakt. Während der Therapie durchströmen die Dialysierflüssigkeit und das Blut des Patienten einen Dialysator und sind innerhalb des Dialysators lediglich durch eine semipermeable Membran voneinander getrennt. Dort findet ein Übergang der während der Dialysetherapie aus dem Blut zu entfernenden Stoffe, wie z.B. Harnstoff vom Blut über die semipermeable Membran in die Dialysierflüssigkeit statt. Klinische Studien zeigen, dass Kontaminationen im Dialysewasser zu akuten und chronischen Problemen beitragen und zu schweren Komplikationen bei Hämodialysepatienten führen können, wenn diese bspw. in das Blut des Patienten gelangen. Daher ist die Wasserqualität ein entscheidender Schlüsselfaktor der modernen Dialyse.

Aus der US 6,797,173 B1 sind eine Vorrichtung und ein Verfahren, welche für ein Umkehrosmosesystem geeignet sind, bekannt. Dabei ist eine Prozesskammer mit einem Einlass, einem Niederdruckauslass und einem Hochdruckauslass vorgesehen. Eine Förderpumpe wird verwendet, um den Zufuhrdruck für die Prozesskammer zu erhöhen.

Nachteilig bei bekannten Umkehrosmoseanlagen ist, dass diese ohne Berücksichtigung der aktuellen Umgebungsbedingen, des Materialzustandes und des Zustandes der angeschlossenen Geräte betrieben werden.

Der Erfindung liegt daher die Aufgabe zugrunde, ein Verfahren zum Betreiben einer Umkehrosmoseanlage bereitzustellen, welches einen optimierten Betrieb einer konkret installierten Umkehrosmoseanlage ermöglicht und die Nachteile aus dem Stand der Technik beseitigt.

Diese Aufgabe wird erfindungsgemäß gelöst durch ein Verfahren mit den Merkmalen von Anspruch 1. Das Verfahren zum Betreiben einer Umkehrosmoseanlage, welche wenigstens eine Druckerhöhungspumpe, wenigstens ein Membranmodul und eine Ringleitung mit wenigstens einer Abnahmestelle umfasst, weist die folgenden Schritte auf:
a) Selektieren wenigstens eines anlagenspezifischen Parameters der Umkehrosmoseanlage und Definieren einer optimierten Systemreaktion der Umkehrosmoseanlage in Abhängigkeit von dem wenigstens einen anlagenspezifischen Parameter der Umkehrosmoseanlage;
b) iteratives Verändern des wenigstens einen anlagenspezifischen Parameters der Umkehrosmoseanlage;
c) Messen der Systemreaktion der Umkehrosmoseanlage auf den wenigstens einen veränderten anlagenspezifischen Parameter der Umkehrosmoseanlage;
d) optionales Anpassen des Veränderns des wenigstens einen anlagenspezifischen Parameters der Umkehrosmoseanlage, um eine Systemreaktion zu erreichen, welche näher an der optimierten Systemreaktion liegt;
e) bei Erreichen der optimierten Systemreaktion innerhalb eines vorgegebenen Schwellenbereiches, Festlegen wenigstens eines optimierten Betriebsparameters;
f) Betreiben der Umkehrosmoseanlage mit diesem wenigstens einen optimierten Betriebsparameter.

Vorteilhafte Ausgestaltungen der Erfindung sind Gegenstand der Unteransprüche.

Die Erfindung geht von der Überlegung aus, dass eine Umkehrosmoseanlage möglichst effektiv und effizient betrieben werden sollte. Zugleich sollte sie ressourcenschonend betrieben werden. Zwar kann eine derartige Umkehrosmoseanlage vom Hersteller vor der Auslieferung in gewissen Grenzen optimiert werden. Die konkreten Installationen der Umkehrosmoseanlagen können sich aber unterscheiden, beispielsweise durch ausgetauschte Komponenten, das Alter von Komponenten oder die konkreten Umgebungsbedingungen, sodass die gleiche initiale Konfiguration nicht für alle Umkehrosmoseanlagen optimiert ist.

Wie nunmehr erkannt wurde, kann eine Umkehrosmoseanlage optimiert betrieben werden, indem bei der konkret installierten Umkehrosmoseanlage eine Optimierung von anlagenspezifischen Betriebsparametern durchgeführt wird, sodass die Umkehrosmoseanlage dann mit diesen optimierten Parametern betrieben werden kann. Diese Optimierung der Parameter kann direkt einmalig nach Installation der Anlage erfolgen. Sie kann aber auch bedarfsweise oder in regelmäßigen Abständen erfolgen, sodass technische bzw. materialtechnische Veränderungen oder die Art der angeschlossenen Geräte berücksichtigt werden können.

Vorteilhafterweise wird die Variation des oder der Parameter in einer Geschwindigkeit und/oder Schrittweite durchgeführt, dass sich vor der nächsten Variation der Parameter die Systemreaktion auf die veränderten Parameter vollständig eingestellt hat, sodass die Umkehrosmose in einem (dynamischen) Gleichgewichtszustand befindet, bevor die nächste Variation des oder der Parameter erfolgt.

In einer ersten bevorzugten Ausführungsform des Verfahrens korrespondiert die optimierte Systemreaktion zu einem Schwellenwert wenigstens einer messbaren Größe in der Umkehrosmoseanlage. Eine messbare Größe bezeichnet hier bevorzugt eine mittels wenigstens eines Sensors messbare Systemreaktion, beispielsweise einen Druck in einer Leitung.

Vorteilhafterweise ist ein erster Druck in der Umkehrosmoseanlage oder stromabwärts davon am Anfang der Ringleitung messbar und ein zweiter Druck ist an wenigstens einer Abnahmestelle messbar.

In Schritt a) wird als anlagenspezifischer Parameter die Drehzahl der Druckerhöhungspumpe selektiert, wobei die optimierte Systemreaktion vorliegt, wenn der erste Druck zuerst einen ersten Druckschwellenwert erreicht oder wenn der zweite Druck zuerst einen zweiten Druckschwellenwert erreicht, und wobei
in Schritt b) die Drehzahl der Druckerhöhungspumpe iterativ erhöht wird, und wobei in Schritt c) der erste Druck und der zweite Druck gemessen werden, und wobei in Schritt e) bei Erreichen der optimierten Systemreaktion innerhalb des vorgegebenen Schwellenbereiches, d.h. bei Erreichen eines der beiden Druckschwellwerte, der aktuelle Druck als maximaler Druckschwellenwert der Umkehrosmoseanlage am Anfang der Ringleitung festgelegt wird. Sobald einer der beiden Schwellwerte erreicht wird, wird somit der Druck am Anfang der Ringleitung als neuer Schwellwert gesetzt.

Der Anfang der Ringleitung bezeichnet hierbei den Bereich der Ringleitung, in den das Permeat eintritt, bevor es durch die Ringleitung zu den Abnahmestellen fließt. An den beiden Orten werden jeweils die Drücke gemessen, während die Druckerhöhungspumpe ihre Drehzahl erhöht. Sobald eine der beiden Druckgrenzen erreicht wurde, ist der Vorgang beendet. Der optimierte Wert, welcher sich aus diesem Verfahren ergibt, wird auf den Drucksensor angewendet und dort gemessen.

Wenn der Druckschwellenwert zuerst an einem Drucksensor am Anfang der Ringleitung erreicht wird, wird dieser gemessene Druck als maximaler Druckschwellenwert verwendet. Wenn der Druckschwellenwert an der Abnahmestelle zuerst den Druckschwellenwert erreicht, wird der zu diesem Zeitpunkt vorliegende Druck am Anfang der Ringleitung als maximaler Druckschwellenwert verwendet.

Auf diese Weise kann sichergestellt werden, dass die Umkehrosmoseanlage mit einem maximalen Druck betrieben werden kann, welcher durch den maximal erlaubten Druck für Komponenten der Umkehrosmoseanlage oder angeschlossene Geräte bestimmt wird.

In einer zweiten bevorzugten Ausführung des Verfahrens korrespondiert die optimierte Systemreaktion zu einem Extremum, insbesondere Minimum, eines von den anlagenspezifischen Parametern abhängigen Fehlermaßes. Ein Minimum wird insbesondere dann definiert, wenn das Fehlermaß als Summe von positivdefiniten Summanden definiert wird.

Vorteilhafterweise weist die Umkehrosmoseanlage wenigstens einen Regelkreis mit wenigstens einem Regler mit wenigstens einem Reglerparameter auf, wobei in Schritt a) als anlagenspezifische Parameter charakteristische Größen des Reglers selektiert werden, und wobei die optimierte Systemreaktion zu einem Minimum eines Fehlermaßes korrespondiert, welches von diesen charakteristischen Größen abhängt, und wobei in Schritt b) in einer Iteration wenigstens eine der charakteristischen Größen verändert wird, und wobei in Schritt c) aufgrund der Systemreaktion das Fehlermaß bestimmt wird, und wobei in Schritt e) bei Erreichen der optimierten Systemreaktion innerhalb des vorgegebenen Schwellenbereiches der wenigstens eine aktuelle Reglerparameter als optimierter Regelparameter festgelegt wird.

Vorteilhafterweise wird Schritt d) ausgeführt, indem zur Veränderung wenigstens einer charakteristischen Größe in den Schritten b) und d) ein Gradientenschrittverfahren ausgeführt wird. Der Gradient in einem Parameterraum korrespondiert zu der Richtung im Parameter mit der größten Änderung. Dadurch bewegt sich das Verfahren durch Folgen des Gradienten schnell zu dem Extremum. Es ist dadurch deutlich effektiver als andere Optimierungsverfahren, bei denen die Parameter im Wesentlichen unabhängig voneinander variiert werden.

In dem Gradientenschrittverfahren wird vorteilhafterweise eine variable und von der Änderungsrate des Fehlermaßes abhängige Schrittweite für die iterativ veränderten Parameter verwendet.

In einer bevorzugten Ausführungsform ist das das Fehlermaß eine gewichtete Summe der charakteristischen Größen. Die Gewichtung wird bevorzugt durch Multiplikation der jeweiligen charakteristischen Größe mit eine Skalenfaktor realisiert, wodurch auch eine Anpassung an eine Summe von Größen mit unterschiedlichen Einheiten erfolgen kann.

Bevorzugt sind die charakteristischen Größen die Anstiegszeit, die Einschwingzeit und das Über-/Unterschwingen. Bei der Anstiegs- und Einschwingzeit werden dabei bevorzugt die tatsächlichen Zeiten vom Start der Messung bis zum Erreichen des Endpunktes gemessen. Bei der Anstiegszeit wird dabei bevorzugt die Zeit gestoppt, welche benötigt wird, um von 10% auf 90% des finalen Wertes / Endwertes zu steigen. Bei der Einschwingzeit wird bevorzugt die Zeit gestoppt, welche benötigt wird, damit der Prozesswert den Endwert innerhalb vorgegebener Grenzen (beispielsweise +/- 2%) erreicht. Dieser Wert "bestraft" Regler die eine niedrige Dämpfung haben, da diese Regler tendenziell stärker Schwingen.

Der wenigstens eine Regler ist vorzugsweise ein PI-Regler, wobei die Reglerparameter Kp und Ti sind.

In einer bevorzugten Ausführungsform des Verfahrens ist der Verfahrensschritt d des Anpassen des Veränderns des wenigstens einen anlagenspezifischen Parameters der Umkehrosmoseanlage, um eine Systemreaktion zu erreichen, welche näher an der optimierten Systemreaktion liegt, ein zwingender Verfahrensschritt, welcher insbesondere iterativ mit den Verfahrensschritt b und c durchgeführt wird. Das bedeutet insbesondere, dass jeweils eine Iteration des Verfahrens die Abfolge der Schritte b, c und d umfasst, wobei in Schritt d beispielsweise die Schrittweite des Veränderns des Parameters verändert wird, wobei diese Abfolge beendet wird bei Verfahrensschritt e des Erreichens der optimierten Systemreaktion innerhalb eines vorgegebenen Schwellenbereiches und des Festlegen wenigstens eines optimierten Betriebsparameters und/oder Schwellenwertes. Auf diese Weise wird das besonders zielgerichtete und effektive Auffinden des zur optimierten Systemreaktion gehörigen optimierten Betriebsparameters und/oder Schwellenwertes ermöglicht.

In einer vorteilhaften Ausführungsform des Verfahrens, werden die Verfahrensschritte a bis e in einem Vorbetrieb und/oder Zwischenbetrieb durchgeführt werden, in welchem an keiner der Abnahmestellen ein Verbraucher Flüssigkeit entnimmt. Dieser Vorbetrieb/Zwischenbetrieb ist deshalb relevant, da auf diese Weise das zeitinvariante System, vor allem Umkehrosmoseanlage und Ringleitung, ohne Störgrößen (Dialysegeräte oder sonstige Verbraucher) bewertet und vermessen werden können. Dies ist vorteilhaft, da die Störgrößen ansonsten als Teil der Systemcharakteristik gewertet werden würden, was die wenigstens eine Iteration des Verfahrens verfälschen würde.

Insbesondere werden vorzugsweise die Verfahrensschritte a bis e in einem Vorbetrieb, beispielsweise vor der allerersten Inbetriebnahme, durchgeführt, bevor der die Umkehrosmose regulär, d.h. mit an den Abnahmestellen angeschlossenen Verbrauchern, betrieben wird. Dadurch kann von Anfang an ein optimierter Betrieb der Umkehrosmoseanlage erreicht werden.

Die Verfahrensschritte a bis e werden weiterhin bevorzugt in einem Zwischenbetrieb in regelmäßigen Abständen und/oder bedarfsweise durchgeführt. Das heißt, der reguläre Betrieb der Umkehrosmoseanlage wird unterbrochen, und die Schritte a bis e werden durchgeführt. Auf diese Weise können Veränderungen der Umkehrosmose, die im Laufe der Zeit auftreten und die zu veränderten optimierten Betriebsparametern führen können, berücksichtigt werden. Veränderungen, welche über die Zeit hinweg an der Umkehrosmoseanlage entstehen, sind beispielsweise eine Wandlung der Systemcharakteristik der Membranen. Diese können durch verblockungs- und/oder mikrobakterielle Erscheinungen einen höheren Druckverlust verursachen, was wiederum die Systemdynamik beeinflusst. Ebenfalls sind Verschleißerscheinungen durch Komponenten und/oder geänderte Fluiddynamiken durch Ablagerungen in dem Gerät nennenswerte Beispiele für eine Transformation des eigentlich statischen Systems.

Vorteilhafterweise wird in Schritt c zum Messen der Systemreaktion ein Testimpuls auf die Umkehrosmoseanlage gegeben. Der Testimpuls dient dazu, die Umkehrosmose in einen Systemzustand zu bringen, welcher die Bestimmung der entsprechenden (stationären) Systemreaktion ermöglicht.

Bei dem Testimpuls kann die Umkehrosmoseanlage aus dem Aus-Zustand oder einem ruhenden Zustand gestartet und auf den Wert des anlagenspezifischen Parameter eingestellt werden, wobei der Testimpuls beendet wird, wenn die Systemreaktion einen stationären Endwert erreicht hat oder nach einer vordefinierten Zeit abgelaufen ist.

Während des Testimpulses kann sich auch die Umkehrosmose in einem aktiven Zustand befinden bzw. der Testimpuls wird im aktiven Zustand der Umkehrosmoseanlage durchgeführt. Im Rahmen des Testimpulses wird dann der Wert des anlagenspezifischen Parameter eingestellt, wobei der Testimpuls beendet wird, wenn die Systemreaktion einen stationären Endwert erreicht hat oder nach einer vordefinierten Zeit abgelaufen ist.

Die Testimpulse werden vorzugsweise während des oben beschriebenen Vorbetriebs und/oder Zwischenbetriebs durchgeführt werden.

Die Steuer- und Regeleinheit der Umkehrosmoseanlage weist vorteilhaft eine Schnittstelle auf, welche die Einstellung der Skalierungsfaktoren und des Fehlermaßes durch einen Nutzer erlaubt, um die örtlichen Anforderungen an den Regelkreis mit einzubinden. Dazu kann die Steuer- und Regeleinheit eine Benutzerschnittstelle in Form eines GUI und/oder eine Datenschnittstelle aufweisen.

Das Verfahren wird in einer bevorzugten Ausführungsform in regelmäßigen Abständen durchgeführt, sodass Veränderungen der Umkehrosmoseanlage und/oder der angeschlossenen Geräte berücksichtigt werden können.

Die Vorteile der Erfindung liegen insbesondere darin, dass durch das Verfahren ein an die konkret vorliegenden Bedingungen und Anforderungen optimierter Betrieb einer Umkehrosmoseanlage ermöglicht wird.

Ein Ausführungsbeispiel der Erfindung wird anhand einer Zeichnung näher erläutert. Darin zeigen in stark schematisierter Darstellung:
- FIG. 1: eine Umkehrosmoseanlage in einer bevorzugten Ausführungsform;
- FIG. 2: ein Ablaufdiagramm eines Verfahrens zum Betreiben einer Umkehrosmoseanlage in einer ersten bevorzugten Ausführungsform;
- FIG. 3: ein Ablaufdiagramm eines Verfahrens zum Betreiben einer Umkehrosmoseanlage in einer zweiten bevorzugten Ausführungsform, und
- FIG. 4: ein Fehlermaß des Verfahrens gemäß FIG. 3.

Gleiche Teile sind in allen Figuren mit denselben Bezugszeichen versehen.

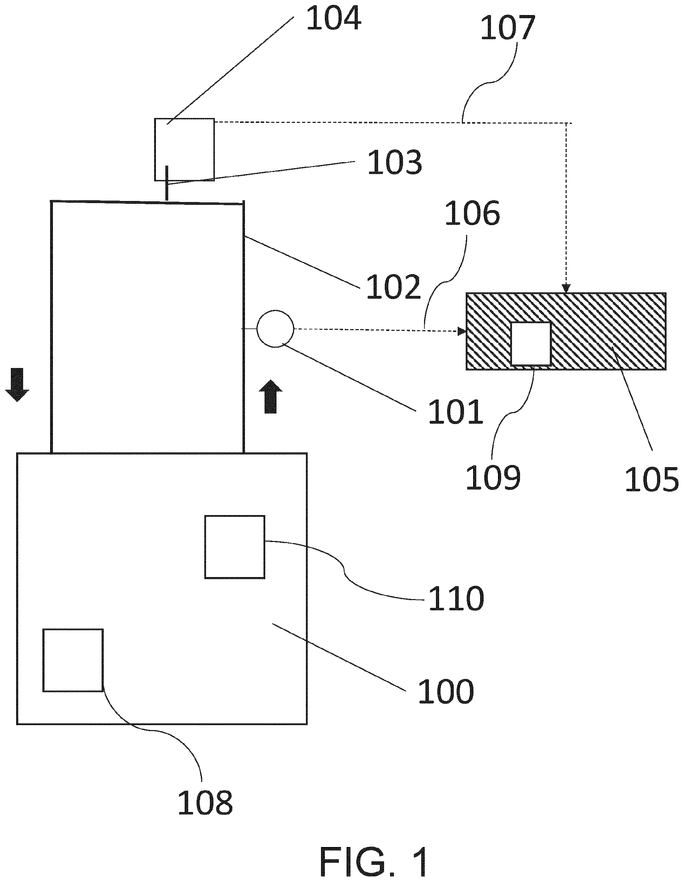

In FIG. 1 ist eine Umkehrosmoseanlage 100 dargestellt, an welcher hydraulisch eine Ringleitung 102 mit einer ersten Abnahmestelle 103 angeschlossen ist. Die Umkehrosmoseanlage 100 weist eine Steuer- und Regeleinheit 105 auf. Die Steuer- und Regeleinheit 105 ist bevorzugterweise eine Steuer- und Regeleinheit 105, welche in der Umkehrosmoseanlage 100 verbaut ist.

Mit der Ringleitung 102 ist ein Drucksensor 101, welcher den Druck in der Ringleitung 102 misst, hydraulisch verbunden. Der Drucksensor 101 ist signaleingangsseitig mit der Steuer- und Regeleinheit 105 durch eine Datenleitung 106 verbunden. An der ersten Abnahmestelle 103, welche sich stromabwärts der Umkehrosmoseanlage 100 befindet, ist ein Testgerät 104 angeschlossen, welches ebenfalls signaleingangsseitig mit der Steuer- und Regeleinheit 105 durch eine Datenleitung 107 verbunden ist. Die Steuer- und Regeleinheit 105 weist einen PI-Regler 109 auf, welcher ausgebildet ist, die Umkehrosmoseanlage 100, insbesondere ihre Pumpe(n), aufgrund von erfassten Sensordaten in einem Regelkreis zu regeln.

Die Umkehrosmoseanlage 100 weist wenigstens ein Membranmodul 110 zur Filterung von Flüssigkeit auf. Die gefilterte Flüssigkeit, das Permeat wird im Membranmodul 110 von Konzentrat getrennt. Die Umkehrosmoseanlage 100 fördert das Permeat in die Ringleitung 102. Sie weist dazu eine Druckerhöhungspumpe 108 auf.

Der Drucksensor 101 ist entweder in der Umkehrosmoseanlage 100 (nicht dargestellt) oder, wie in FIG. 1 dargestellt, stromabwärts davon am Anfang der Ringleitung 102 angeordnet. Das Testgerät 104 besteht aus wenigstens einer Kupplung zum Anschließen an die erste Abnahmestelle 103 und einem Drucksensor.

Der maximale Druck im Ringleitungsvorlauf wird essenziell von zwei Faktoren beeinflusst. Der erste Faktor ist das Ringleitungsmaterial. Beispielsgemäß erlaubt das Material PEX einen maximalen Druck von 10 bar, während Edelstahl einen maximalen Druck von 25 bar erlaubt, wobei im Allgemeinen derartige Druckmaximalwerte materialstärkenabhängig und temperaturabhängig sind.

Ein Dialysegerät, welches beispielsweise an der ersten Annahmestelle 103 angeschlossen ist, ist der zweite Faktor und erlaubt beispielsweise einen maximalen Druck von 6 bar. Dialysegeräte erlauben somit nur einen niedrigeren Druck als die entsprechende Ringleitung. Allerdings ist nicht fest definiert, wo das erste Dialysegerät positioniert ist. Das bedeutet, dass der Druckverlust bis zur ersten Abnahmestelle 103 auf 6 bar beaufschlagt werden kann.

Das erfindungsgemäß Verfahren wird eingesetzt, um einen maximalen Schwellenwert für den Ringleitungsdruck zu bestimmen.

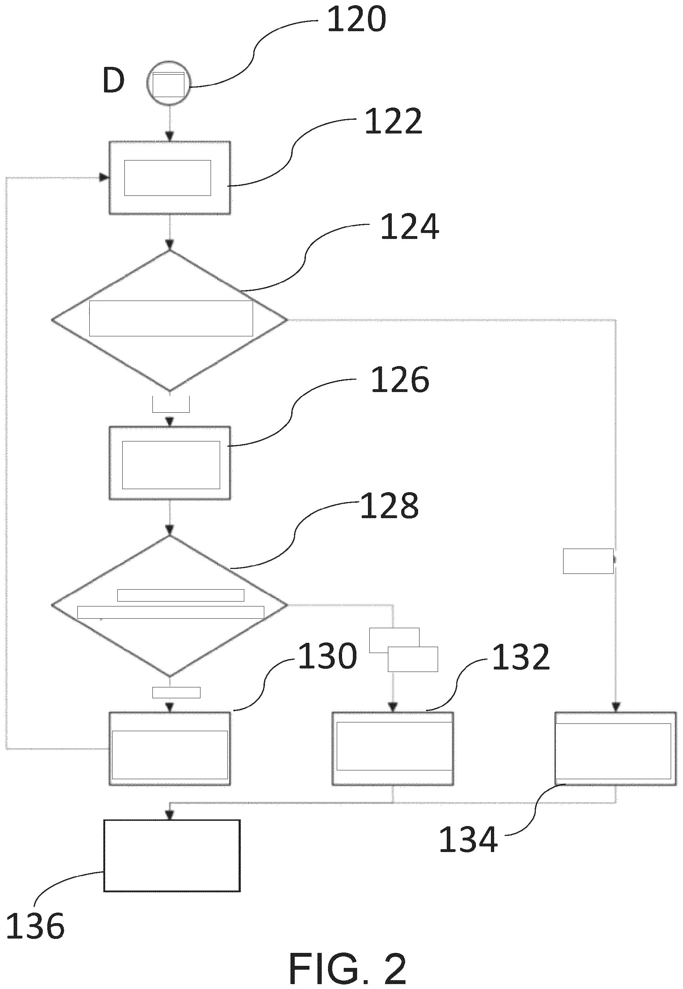

Das in FIG. 2 dargestellte Verfahren startet bei einem Start 120. Als anlagenspezifischer Parameter wird die Drehzahl D der Druckerhöhungspumpe 108 selektiert. Es wird eine optimierte Systemreaktion der Umkehrosmoseanlage in Abhängigkeit von dieser Drehzahl definiert. Vorliegend liegt eine optimierte Systemreaktion vor, wenn der erste Druck zuerst einen ersten Druckschwellenwert erreicht oder wenn der zweite Druck zuerst einen zweiten Druckschwellenwert erreicht.

In einem Block 122 wird der Vordruck in der Ringleitung 102 ausgewertet.

In einer Entscheidung 124 wird überprüft, ob der maximale Vordruck erreicht ist.

Ist dies nicht der Fall, wird in einem Block 126 der von dem Testgerät 104 gemessene Druck ausgewertet.

In einer Entscheidung 128 wird überprüft, ob der vom Testgerät 104 gemessene Druck den maximalen Dialysemaschinendruck (im vorliegenden Beispiel 6 bar) erreicht hat.

Ist der maximale Dialysemaschinendruck nicht erreicht, wird in einem Block 130 die Drehzahl D der Druckerhöhungspumpe 108 erhöht und wieder in Block 122 weitergeführt. Zur Erhöhung der Pumpendrehzahl wird die Druckerhöhungspumpe 108 von der Steuer- und Regeleinheit 105 angesteuert.

Sofern der maximale Dialysemaschinendruck erreicht ist, geht das Verfahren weiter in einem Block 132. Es liegt die optimierte Systemreaktion vor. Der Druckschwellenwert wird auf den aktuellen Druckwert des Drucks in der Ringleitung 102 gesetzt, welcher vom Drucksensor 101 gemessen wird. Das Verfahren wird in einem Block 136 weitergeführt, in dem die Umkehrosmoseanlage 100 mit dem bestimmten Druckschwellenwert PS betrieben wird.

Sofern in der Entscheidung 124 die Überprüfung ergeben hat, dass der maximale Ringdruck erreicht ist, verzweigt das Verfahren zu einem Block 134, in welchem der Druckschwellenwert auf den vom Drucksensor 101 gemessenen maximalen Ringleitungsdruck gesetzt wird. Es liegt die optimierte Systemreaktion vor. Das Verfahren wird in einem Block 136 weitergeführt, in dem die Umkehrosmoseanlage 100 mit dem bestimmten Druckschwellenwert PS betrieben wird.

Zusammengefasst erhöht die Druckerhöhungspumpe 108 der Umkehrosmoseanlage 100 sukzessive die Drehzahl D der Druckerhöhungspumpe 108, bis entweder der Drucksensor 101 den Druck von 10 bar erreicht oder das Testgerät 104 den Druck von 6 bar erreicht

Die genannten Druckwerte 6 bar und 10 bar basieren auf den definierten Interfaces bzw. erlaubten anschließbaren Geräten. Die Drücke sind also als verdeutlichendes Beispiel und nicht als allgemein gültige Werte zu verstehen. Das im Zusammenhang mit FIG. 2 beschriebene Verfahren wird während der Schwellwertermittlung mit ausreichender Abtastrate durchgeführt bzw. die sukzessive Pumpendrehzahlerhöhung wird entsprechend langsam durchgeführt, damit der Druckschwellenwert möglichst exakt bestimmt wird. Daneben können auch Sicherheitsgrenzwerte, z.B. 0,2 bar unter dem ermittelten Wert, angewendet werden, sodass das System Messtoleranzen ausgleichen kann. Auf diese Weise kann auch das Überschwingen des Reglers bzw. eine Druckspitze sicherheitsgerichtet berücksichtigt werden.

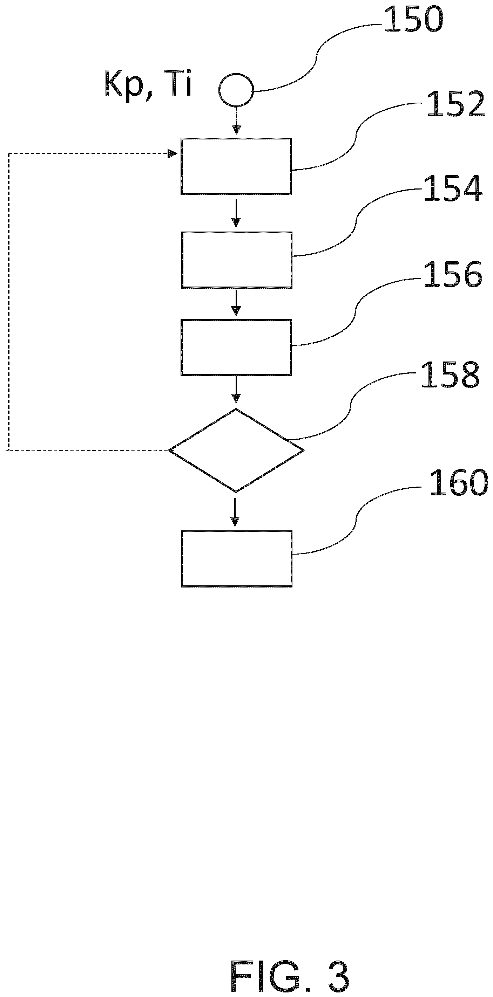

Ein Verfahren in einer zweiten bevorzugten Ausführungsform wird im Zusammenhang mit FIG. 3 beschrieben, in welchem optimierte Regelparameter des PI-Reglers 109 bestimmt werden.

Regelkreise funktionieren optimal, wenn sie schnell auf Änderungen reagieren und ein geringes Über/Unterschwingverhalten aufweisen. Je nachdem, welcher Regler-Typ (PID, PI2D, PI, PD, State Space etc.) bzw. welche Systemkomplexität vorhanden ist, gibt es eine Vielzahl an Parametern, welche das Verhalten beeinflussen.

Durch das schrittweise Verändern eines oder mehrere Parameter und des Messens der optimierten Systemreaktion kann eine inkrementelle Verbesserung/ Optimierung der Regelung der Umkehrosmoseanlage 100 durch den PI-Regler 109 erzielt werden.

Der PI-Regler 109 weist zwei veränderliche Parameter auf, nämlich Kp und Ti. Hierbei ist Kp im bekannten Sinne die Reglerverstärkung, die spezifiziert, wie stark der PI-Regler auf die Regeldifferenz reagiert, und Ti die Nachstellzeit, welche die Zeit bezeichnet, die verstreichen muss, damit der I-Anteil gleich dem P-Anteil ist. Das Verfahren ist auch auf Systeme höherer oder niedrigere Komplexität, in Hinsicht auf Parameter/Freiheitsgrade, anwendbar. Die Veränderung der Parameter Kp und Ti beeinflusst die Charakteristik des PI-Reglers 109.

Im vorliegenden Ausführungsbeispiel werden drei Eigenschaften bzw. charakteristische Größen, welche die Charakteristik des PI-Reglers 109 beeinflussen, ausgewählt, nämlich die Anstiegszeit [in Sekunden], die Einschwingzeit [in Sekunden] und Über-/Unterschwingen [in Prozent].

Aus diesen Eigenschaften wird ein Fehlermaß J generiert, welches zur Gütebewertung der Charakteristik verwendet wird. Das Fehlermaß J wird berechnet gemäß

J = a * Anstiegszeit + b * Einschwingzeit + c * Über-/Unterschwingen ("*" steht für Multiplikation).

Die Faktoren a, b, und c sind Skalierungsfaktoren, mit welchen die jeweilige Eigenschaft im Fehlermaß J priorisiert werden kann. Mit den Skalierungsfaktoren bzw. /Prioritätszahlen a, b, c kann somit der Fehler auf Wichtigkeit (z. B. Anstiegszeit wichtiger als Einschwingzeit) getrimmt werden. Außerdem können verschieden dimensionierte Eigenschaften (hier Prozent und Sekunden) so skaliert werden, dass diese die Wichtigkeit für die Gesamtcharakteristik reflektieren.

Bei einem Start 150 werden als anlagenspezifische Parameter die oben genannten Parameter Kp und Ti des Reglers selektiert. Die optimierte Systemreaktion wird zu einem Minimum des oben genannten Fehlermaßes J, welches von diesen charakteristischen Größen abhängt, welche wiederum von den Parametern Kp und Ti abhängen, definiert.

In einem Block 152 wird wenigstens einer der Parameter Kp, Ti variiert, und es wird ein Testimpuls auf das System der Umkehrosmoseanlage 100 gegeben. Bei dem Testimpuls wird das System auf dem Aus-Zustand bzw. einem ruhenden Zustand gestartet und auf den Sollwert eingeregelt. Sobald der Prozesswert den stationären Endwert erreicht hat, ist der Testimpuls beendet. In einer alternativen Ausführung des Testimpulses kann das System bereits aktiv sein und der Sollwert wird verändert.

In dem gezeigten Beispiel wird ein Sprung im Sollwert genutzt, um das Fehlermaß zu bestimmen. Das System "ruht" zunächst (Regelausgang=0%; Sollwertabweichung=0). Anschließend soll der Sollwert erreicht werden (z.B. 3 bar). Durch die sprunghafte Veränderung des Sollwerts erhöht sich die Sollwertabweichung und die Regelcharakteristik kann beobachtet werden.

In bevorzugter Weise werden nicht beide Parameter nach einem statischen Prinzip in-/dekrementiert, da dies die Dauer der Parameterfindung erhöht.

Das Variieren der beiden Parameter Kp, Ti erfolgt vorteilhafterweise mit einem Gradientenschrittverfahren. Dabei wird automatisch eine Konvergenz zu einem Minimum erreicht. Zusätzlich zum Gradientenschrittverfahren wird in bevorzugter Ausführungsform eine adaptive Schrittweitenregelung angewendet, welche es erlaubt, das Finden des Minimums zu beschleunigen. Dabei wird anstelle einer festen In-/ Dekrementierung der Regelwerte die Schrittweite der Parameter an die Änderungsrate des Fehlermaßes angepasst. Somit wird bei kleinen Veränderungen des Fehlermaßes ebenfalls nur noch kleine Änderungen an den Parametern vorgenommen, wohingegen bei großen Veränderungen des Gütemaßes ebenfalls große Änderungen an den Parametern vorgenommen werden.

In einem Block 154 werden die Anstiegszeit, die Einschwingzeit und das Über-/Unterschwingen gemessen. Aus diesen Größen wird nach der oben dargestellten Formel das Fehlermaß J in einem Block 156 berechnet.

In einer Entscheidung 158 wird überprüft, ob ein vorgegebenes Kriterium für die Erreichung des Minimums erreicht ist, was das Erreichen der optimierten Systemreaktion innerhalb des vorgegebenen Schwellenbereiches ist, keine Verringerung des Fehlers mehr möglich ist oder die Änderungsrate des Fehlers kleiner als ein vorgegebener Schwellwert ist.

Ist dies nicht der Fall, verzweigt das Verfahren wieder zu Block 152. Ist das Kriterium erfüllt, geht das Verfahren weiter zu einem Block 160, in welchem die aktuellen Reglerparameter als optimierte Regelparameter festgelegt werden und die Umkehrosmoseanlage 100 mit den optimierten Werten der Reglerparameter Kp, Ti betrieben wird.

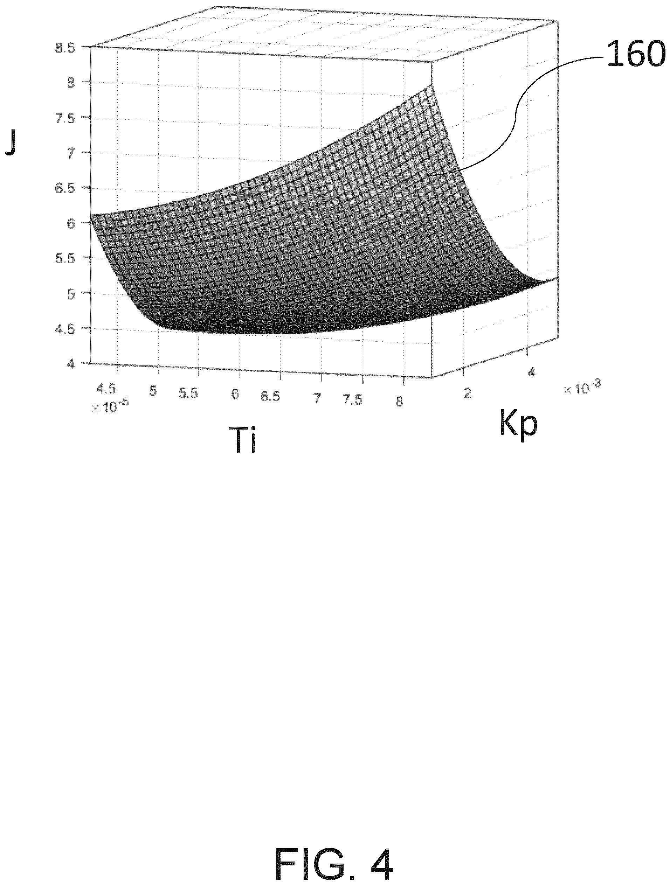

In FIG. 4 ist beispielhaft das Fehlermaß J gegen die Regelparameter Ti und Kp in einer interpolierten Darstellung aufgetragen (der Parameterraum wird in diskreten Schritten abgetastet). Dabei ergibt sich ein Paraboloid, welcher ein Minimum aufweist. Um die optimalen Regelparameter zu ermitteln, kann das Minimum des Paraboloids berechnet werden. Dieser gibt die optimalen Regelparameter für das definierte Fehlermaß J an. In einer bevorzugten Ausführungsform des Verfahrens wird das Fehlermaß J gemittelt. Bevorzugt wir dazu die Quantität der Testzyklen bei gleichem Parametersatz erhöht (z.B. drei Testzyklen bei den gleichen Werten) und der Mittelwert über diese Messungen gebildet.

Da die Bedingungen zur Ermittlung des Sehwellwertes dynamisch von den angeschlossenen Geräten und Umgebungsbedingungen abhängig sind, sollte der Nutzer die Möglichkeit haben, die Bedingungen zu definieren. Dies kann entweder über eine Eingabe von Werten oder einer Auswahl von erlaubten Geräten über ein Enum (Edelstahlringleitung = 25 bar; PEX = 10 bar; Dialysegerät = 6 bar; etc.) geschehen.

Anstelle des Drucksensors 101 gemäß FIG. 1 kann auch ein parametrierbarer Drucksensor mit Schaltercharakteristik verwendet werden. Dabei soll der Schalter über z. B. über einen IO-Link von der Steuer- und Regeleinheit 105 auf den ermittelten Wert gesetzt werden.

Das Testgerät 104 gemäß FIG. 1 kann auch ein Dialysegerät oder sonstiger regulärer Abnehmer sein, welcher mithilfe einer Netzwerkverbindung den Druck am Wassereingang an die Umkehrosmoseanlage 100 bzw. an die Steuer- und Regeleinheit 105 überträgt oder auch die entsprechenden Daten an eine Cloud überträgt, auf die von der Steuer- und Regeleinheit 105 zugegriffen werden kann.

Das oben beschriebene Verfahren umfasst eine einmalige Ausführung zum Ermitteln der optimierten Parameterwerte. Die Ermittlung von Schwellwerten kann durch Netzwerktechnologie auch iterativ jede Stunde, jeden Tag, jeden Monat durchgeführt werden. Dies hat den Vorteil, dass bei einem Umbau des Gesamtsystems, d.h. örtliche Veränderung der ersten Abnahmestelle 103, der Schwellwert ebenfalls mitangepasst wird. Dies bedingt einer Netzwerkanbindung inkl. Druckmessung aller aktiven Entnahmestellen im System.

Die Prioritätszahlen bzw. Skalenfaktoren und die Erstellung des Fehlermaßes bzw. Gütemaßes sollten vom Nutzer beeinflusst werden können, um die örtlichen Anforderungen an den Regelkreis mit einzubinden.

### Bezugszeichenliste

- 100: Umkehrosmoseanlage
- 101: Drucksensor
- 102: Ringleitung
- 103: erste Abnahmestelle
- 104: Testgerät
- 105: Steuer- und Regeleinheit
- 106: Datenleitung
- 107: Datenleitung
- 108: Druckerhöhungspumpe
- 109: PI-Regler
- 110: Membranmodul
- 120: Start
- 122: Block
- 124: Entscheidung
- 126: Block
- 128: Entscheidung
- 130: Block
- 132: Block
- 134: Block
- 136: Block
- 150: Start
- 152: Block
- 154: Block
- 156: Block
- 158: Entscheidung
- 160: Paraboloid

- J: Fehlermaß
- a: Skalierungsfaktor
- b: Skalierungsfaktor
- c: Skalierungsfaktor
- D: Drehzahl
- PS: Druckschwellenwert
- Kp: Reglerverstärkung
- Ti: Nachstellzeit

## Patentansprüche

1. Verfahren zum Betreiben einer Umkehrosmoseanlage (100), welche wenigstens eine Druckerhöhungspumpe (108), wenigstens ein Membranmodul (110) und eine Ringleitung (102) mit wenigstens einer Abnahmestelle (103) umfasst,
**gekennzeichnet durch die Schritte**
a) Selektieren wenigstens eines anlagenspezifischen Parameters (D, Kp, Ti) der Umkehrosmoseanlage (100) und Definieren einer optimierten Systemreaktion der Umkehrosmoseanlage (100) in Abhängigkeit von dem wenigstens einen anlagenspezifischen Parameter (D, Kp, Ti) der Umkehrosmoseanlage;
b) iteratives Verändern des wenigstens einen anlagenspezifischen Parameters (D, Kp, Ti) der Umkehrosmoseanlage (100);
c) Messen der Systemreaktion der Umkehrosmoseanlage (100) auf den wenigstens einen veränderten anlagenspezifischen Parameter (D, Kp, Ti) der Umkehrosmoseanlage;
d) optionales Anpassen des Veränderns des wenigstens einen anlagenspezifischen Parameters (D, Kp, Ti) der Umkehrosmoseanlage (100), um eine Systemreaktion zu erreichen, welche näher an der optimierten Systemreaktion liegt;
e) bei Erreichen der optimierten Systemreaktion innerhalb eines vorgegebenen Schwellenbereiches, Festlegen wenigstens eines optimierten Betriebsparameters (D, Kp, Ti) und/oder Schwellenwertes (PS, J);
f) Betreiben der Umkehrosmoseanlage (100) mit diesem wenigstens einen optimierten Betriebsparameter und/oder Schwellenwert (PS, J).

2. Verfahren nach Anspruch 1, wobei die optimierte Systemreaktion zu einem Schwellenwert (PS, J) wenigstens einer messbaren Größe in der Umkehrosmoseanlage korrespondiert.

3. Verfahren nach Anspruch 2, wobei ein erster Druck in der Umkehrosmoseanlage oder stromabwärts davon am Anfang der Ringleitung (102) messbar ist, und wobei ein zweiter Druck an wenigstens einer Abnahmestelle (103) messbar ist, und wobei
in Schritt a) als anlagenspezifischer Parameter die Drehzahl (D) der Druckerhöhungspumpe (108) selektiert wird, und wobei die optimierte Systemreaktion vorliegt, wenn der erste Druck zuerst einen ersten Druckschwellenwert erreicht oder wenn der zweite Druck zuerst einen zweiten Druckschwellenwert erreicht, und wobei
in Schritt b) die Drehzahl (D) der Druckerhöhungspumpe (108) iterativ erhöht wird, und wobei
in Schritt c) der erste Druck und der zweite Druck gemessen werden, und wobei in Schritt e) bei Erreichen der optimierten Systemreaktion innerhalb des vorgegebenen Schwellenbereiches der aktuelle Druck als maximaler Druckschwellenwert (PS) der Umkehrosmoseanlage (100) am Anfang der Ringleitung (102) festgelegt wird.

4. Verfahren nach Anspruch 1, wobei die optimierte Systemreaktion zu einem Extremum, insbesondere Minimum, eines von den anlagenspezifischen Parametern abhängigen Fehlermaßes (J) korrespondiert.

5. Verfahren nach Anspruch 4, wobei die Umkehrosmoseanlage (100) wenigstens einen Regelkreis mit wenigstens einem Regler mit wenigstens einem Reglerparameter (Kp, Ti) aufweist, und wobei
in Schritt a) als anlagenspezifische Parameter charakteristische Größen des Reglers selektiert werden, und wobei die optimierte Systemreaktion zu einem Minimum eines Fehlermaßes (J) korrespondiert, welches von diesen charakteristischen Größen abhängt, und wobei
in Schritt b) in einer Iteration wenigstens eine der charakteristischen Größen verändert wird, und wobei
in Schritt c) aufgrund der Systemreaktion das Fehlermaß (J) bestimmt wird, und wobei
in Schritt e) bei Erreichen der optimierten Systemreaktion innerhalb des vorgegebenen Schwellenbereiches der wenigstens eine aktuelle Reglerparameter (Kp, Ti) als optimierter Regelparameter festgelegt wird.

6. Verfahren nach Anspruch 5, wobei Schritt d) ausgeführt wird, indem zur Veränderung wenigstens einer charakteristischen Größe in den Schritten b) und d) ein Gradientenschrittverfahren ausgeführt wird.

7. Verfahren nach Anspruch 6, wobei in dem Gradientenschrittverfahren eine variable und von der Änderungsrate des Fehlermaßes abhängige Schrittweite für die iterativ veränderten Parameter verwendet wird.

8. Verfahren nach einem der Ansprüche 5 bis 7, wobei das Fehlermaß (J) eine gewichtete Summe der charakteristischen Größen ist.

9. Verfahren nach einem der Ansprüche 5 bis 8, wobei die charakteristischen Größen die Anstiegszeit, die Einschwingzeit und das Über-/Unterschwingen sind.

10. Verfahren nach Anspruch 9, wobei der wenigstens eine Regler ein PI-Regler ist, und wobei die Reglerparameter Kp und Ti sind.

11. Verfahren nach einem der Ansprüche 1 bis 10, wobei der Verfahrensschritt d des Anpassen des Veränderns des wenigstens einen anlagenspezifischen Parameters (D, Kp, Ti) der Umkehrosmoseanlage (100), um eine Systemreaktion zu erreichen, welche näher an der optimierten Systemreaktion liegt, ein zwingender Verfahrensschritt ist, welcher insbesondere iterativ mit den Verfahrensschritten b und c durchgeführt wird.

12. Verfahren nach einem der Ansprüche 1 bis 11, wobei die Verfahrensschritte a bis e in einem Vorbetrieb und/oder Zwischenbetrieb durchgeführt werden, in welchem an keiner der Abnahmestellen (103) ein Verbraucher Flüssigkeit entnimmt.

13. Verfahren nach einem der Ansprüche 1 bis 12, wobei in Schritt c) zum Messen der Systemreaktion ein Testimpuls auf die Umkehrosmoseanlage (100) gegeben wird.

14. Verfahren nach Anspruch 13, wobei bei dem Testimpuls die Umkehrosmoseanlage (100) aus dem Aus-Zustand oder einem ruhenden Zustand gestartet und auf den Wert des anlagenspezifischen Parameter (D, Kp, Ti) eingestellt wird, und wobei der Testimpuls beendet wird, wenn die Systemreaktion einen stationären Endwert erreicht hat oder nach einer vordefinierten Zeit abgelaufen ist.

15. Verfahren nach Anspruch 13, wobei sich während des Testimpulses die Umkehrosmose (100) in einem aktiven Zustand befindet und im Rahmen des Testimpulses der Wert des anlagenspezifischen Parameter (D, Kp, Ti) eingestellt wird, und wobei der Testimpuls beendet wird, wenn die Systemreaktion einen stationären Endwert erreicht hat oder nach einer vordefinierten Zeit abgelaufen ist.
